(19) **Europäisches Patentamt**
**European Patent Office**
**Office européen des brevets**

(11) **EP 1 388 327 B1**

(12) **EUROPEAN PATENT SPECIFICATION**

(45) Date of publication and mention
of the grant of the patent:
**22.02.2012 Bulletin 2012/08**

(51) Int Cl.:
***A61L 27/56*** (2006.01)

(21) Application number: **02769619.4**

(22) Date of filing: **17.05.2002**

(86) International application number:
**PCT/JP2002/004778**

(87) International publication number:
**WO 2002/091955 (21.11.2002 Gazette 2002/47)**

(54) **ARTIFICIAL KIDNEY HAVING FUNCTION OF METABOLIZING PROTEIN AND METHOD OF CONSTRUCTING THE SAME**

KÜNSTLICHE NIERE MIT PROTEINMETABOLISIERUNGSFUNKTION UND VERFAHREN ZU IHRER HERSTELLUNG

REIN ARTIFICIEL PRESENTANT LA FONCTION DE METABOLISME DE PROTEINE ET SON PROCEDE DE FABRICATION

(84) Designated Contracting States:
**DE FR GB IT**

(30) Priority: **17.05.2001 JP 2001147699**

(43) Date of publication of application:
**11.02.2004 Bulletin 2004/07**

(73) Proprietors:
• **KANEKA CORPORATION**
**Osaka-shi, Osaka 530-8288 (JP)**
• **Tabata, Yasuhiko**
**Uji-shi,**
**Kyoto 611-0024 (JP)**
• **Saito, Akihiko**
**Niigata-shi,**
**Niigata 951-8104 (JP)**

(72) Inventors:
• **TABATA, Yasuhiko**
**Uji-shi, Kyoto 611-0024 (JP)**
• **SAITO, Akihiko**
**Niigata-shi, Niigata 951-8104 (JP)**
• **GEJYO, Fumitake**
**Niigata-shi, Niigata 950-2022 (JP)**

• **FURUYOSHI, Shigeo,**
**c/o KANEKA CORPORATION**
**Settsu-shi, Osaka 566-0072 (JP)**
• **YAMASHITA, Kenji,**
**c/o KANEKA CORPORATION**
**Takasago-shi, Hyogo 676-0027 (JP)**

(74) Representative: **HOFFMANN EITLE**
**Patent- und Rechtsanwälte**
**Arabellastraße 4**
**81925 München (DE)**

(56) References cited:
**WO-A-01/24842          WO-A-95/11048**
**WO-A-98/09582          US-A- 5 681 572**
**US-A- 5 686 289**

• **TABATA Y ET AL: "Vascularization effect of basic fibroblast growth factor released from gelatin hydrogels with different biodegradabilities", BIOMATERIALS, ELSEVIER SCIENCE PUBLISHERS BV., BARKING, GB LNKD- DOI: 10.1016/S0142-9612(99)00121-0, vol. 20, 1 January 1999 (1999-01-01), pages 2169-2175, XP002984643, ISSN: 0142-9612**

## Description

TECHNICAL FIELD

The present invention relates to an artificial kidney having a function of metabolizing protein which comprises, as constituent elements, a sponge sheet with an angiogenesis promoting growth factor-containing hydrogel injected therein as well as cells with megalin expressed on the surface, and to a method of constructing the same.

BACKGROUND ART

In Japan, the treatment of terminal renal insufficiency consists mainly in hemodialysis therapy, since the number of donors for renal transplantation is small. However, the hemodialysis therapy, too, cannot fully-compensate the original renal functions, so that various complications are caused, impairing the quality of life (QOL) and vital prognosis of patients.
It is a weak point of hemodialysis therapy that it cannot compensate the metabolic function for proteins filtrated by glomerular that proximal tubular cells have. Therefore, low-molecular proteins to be originally metabolized by those cells are accumulated in the hemodialysis patient's body and act as uremic toxin proteins, causing various pathogenic conditions. A typical example thereof is dialysis related amyloidosis, which is due to the accumulation of $\beta2$ microglobulin ($\beta$2-m), and it causes osteoarthropathy and/or organ failure. Furthermore, in hemodialysis patients, AGEs, which are modified proteins resulting from glycation, are also accumulated, resulting in arteriosclerosis and/or organ damaging. In recent years, the hemodialysis membranes and hemofiltration techniques have been improved and $\beta$2-m adsorption columns have been put into practical use. However, these have their limits and, therefore, it has been desired that an artificial kidney capable of continuously and satisfactorily compensating the function of metabolizing protein be developed.
To meet such demand, systems utilizing cells have been developed for compensating the function of proximal tubular cells. For example, Humes, HD et al. (Nature Biotech. 17, 451-453 (1999)) reported an artificial kidney which comprises a hemofilter in combination with a hollow fiber module with immortalized proximal tubular epithelial cells caused to adhere onto the hollow fiber inside surface and in which filtered blood is circulated through the lumen containing cultured proximal tubular cells, and Saito, Akira et al. (44th Meeting of the Japanese Society for Dialysis Therapy (1999)) reported a similar artificial kidney (tubules) in which distal tubular cells were used. However, these are all extra-corporeal devices, hence cannot overcome the weak points of extra-corporeal circulation therapy consisting of intermittent blood treatment.
WO 98/09582 describes a prosthetic kidney comprising a porous membrane structure and nephron analogs which can be used in vivo or ex vivo.

SUMMARY OF THE INVENTION

The present invention provides an intra-corporeal artificial kidney having the function of metabolizing protein as well as a method of constructing the same in order to solve the problems discussed hereinabove. The invention further provides an artificial kidney having the function of metabolizing protein by utilizing the functions of megalin which incorporates low-molecular-weight proteins, hormones and so forth into cells by binding thereto.
Thus, the present invention relates to
an intra-corporeal artificial kidney having the function of metabolizing protein
which comprises, as constituent elements, a sponge sheet with an angiogenesis promoting growth factor-containing hydrogel injected therein as well as cells with megalin, which is an endocytosis receptor playing a central role in the protein metabolizing function of proximal tubular cells, as expressed on the surface of the cells, wherein the mean pore size of said sponge sheet is 60 to 400 $\mu$m.
The invention also relates to
a method of constructing an intra-corporeal artificial kidney having the function of metabolizing protein
which comprises using a sponge sheet with an angiogenesis promoting growth factor-containing hydrogel injected therein as well as cells with megalin expressed on the surface, wherein the mean pore size of said sponge sheet is 60 to 400 $\mu$m. Furthermore, the invention relates to
the above method of constructing an artificial kidney
which comprises the step of injecting an angiogenesis promoting growth factor-containing hydrogel into a sponge sheet, and the step of injecting cells with megalin expressed on the surface into the implanted sponge sheet, wherein the sponge sheet is to be implanted subcutaneously.
Furthermore, the invention relates to
the above artificial kidney and the method of constructing the same,
wherein the angiogenesis promoting growth factor is a basic fibroblast growth factor (bFGF);
the bFGF is a human bFGF produced by genetic engineering techniques;
the hydrogel is a gelatin gel;
the gelatin gel is mainly composed of a gelatin species having an isoelectric point of 4.5 to 5.5;
the sponge sheet is made of collagen;
the cells with megalin expressed on the surface are human proximal tubular epithelial cells and/or human proximal tubular epithelial cells with megalin expressed at a high level by means of gene transfer; and/or
the expression of megalin is a result of genetic manipulation.

DETAILED DISCLOSURE OF THE INVENTION

In the following, the present invention is described in detail.

The intra-corporeal artificial kidney having the function of metabolizing protein, according to the invention, comprises, as constituent elements, a sponge sheet with an angiogenesis promoting growth factor-containing hydrogel injected therein as well as cells with megalin expressed on the surface.

As the angiogenesis promoting growth factor to be used in the practice of the invention, there may be mentioned, among others, basic fibroblast growth factor (bFGF), acidic fibroblast growth factor (aFGF), epidermal growth factor (EGF), transforming growth factor-α (TGF-α), vascular endothelial growth factor (VEGF), platelet-derived growth factor-BB (PDGF-BB), and hepatocyte growth factor (HGF).

In the practice of the invention, the above-mentioned angiogenesis promoting growth factor may comprise one single species or a combination of a plurality of species. Furthermore, it is also possible to combinedly use another bioactive factor.

From the viewpoint of more potent growth activity exhibition, bFGF and PDGF-BB, among others, are preferred among the growth factors mentioned above. Furthermore, bFGF, of which wide knowledge as an angiogenesis promoting factor has been accumulated, is one of the growth factors used more preferably.

The bFGF which is to be used in the practice of the invention includes the species extracted from such organs or tissues as the pituitary gland, brain, kidney, adrenal gland, placenta, bone matrix, cartilage, endothelial cells, and fibroblasts, the species produced by such genetic engineering techniques as gene recombination, modifications of these, and other species acting as fibroblast growth factors. Among them, human bFGF species produced by genetic engineering techniques are particularly preferred from the quality and stable supply viewpoint. The modifications mentioned above include, among others, derivatives of bFGF species obtained by extraction or genetic engineering techniques as derived by addition of an amino acid residue or residues to the amino acid sequence of the bFGF, substitution of an amino acid residue or residues for one to some amino acid residues of that sequence, or deletion of one to some amino acid residues of that sequence. In the practice of the invention, it is possible to use these bFGF species singly or in admixture.

In the practice of the invention, the hydrogel is used to maintain the angiogenic effect of the angiogenesis promoting growth factor contained therein by gradually releasing that factor into the surroundings thereof.

The raw material of the hydrogel in the practice of the invention includes, among others, polysaccharides such as cellulose, dextran, agarose, pullulan, starch, hyaluronic acid, alginic acid, chitin and chitosan, and polysaccharide derivatives such as hydroxyethylcellulose and carboxymethylcellulose; polyamino acids such as polyaspartic acid, polyglutamic acid and polylysine; polypeptides such as gelatin, collagen, fibrin and gluten; synthetic polymers having hydrophilic side chain groups, such as polyvinyl alcohol, polyacrylamide, polyvinylpyrrolidone, poly(2-hydroxyethyl methacrylate) , polyvinyl methyl ether, poly(N-vinylacetamide), polyacrylic acid, poly(isobutylene-maleic acid), poly(2-acrylamido-2-methylpropanesulfonic acid), polyacryloxypropanesulfonic acid, polyvinylsulfonic acid, poly(methacryloyloxyethyl-quaternized ammonium chloride), polyvinylpyridine and poly(N,N-dimethyl-N-(2-methacryloyloxyethyl)-N-(3-sulfoprop yl) ammonium internal salt) ; and synthetic polymers whose main chain itself is hydrophilic, such as polyethylene glycol, polydioxolane and polyethyleneimine.

In the practice of the invention, these materials may be used singly, or an appropriate combination of a plurality of them may be used or they may be used after further modification with another compound.

Among the raw materials of the hydrogel mentioned above, such polysaccharides as cellulose, hyaluronic acid, alginic acid, chitin and chitosan, such polyamino acids as polyaspartic acid, polyglutamic acid and polylysine, and such polypeptides as gelatin, collagen and fibrin, among others, are bioabsorbable, hence are preferred. In particular, gelatin is preferably used from the viewpoint of easy processability, of maintenance of the bioactivity of the growth factor contained, and of *in vivo* decomposition rate (gradual release of the growth factor).

Gelatin may be obtained by subjecting living organism-derived collagen to appropriate pretreatment with an acid or alkali, for instance, and then to extraction with heating using warm water. In the practice of the invention, however, any of those grades of gelatin which are generally available can be used without any particular limitation. As such, there may be mentioned alkali-treated gelatin having an isoelectric point of 4.5 to 5. 5, and acid-treated gelatin having an isoelectric point of 8 to 9, for instance. In cases where bFGF is used as the angiogenesis promoting factor, alkali-treated gelatin with an isoelectric point of 4.5 to 5.5 is preferably used from the bFGF retention viewpoint, since bFGF has an isoelectric point of about 9 and is positively charged in neutral aqueous solutions. Furthermore, not only one single gelatin species but also two or more species differing in origin or in physical properties such as solubility, molecular weight and isoelectric point may be used in admixture. Furthermore, gelatin may contain another additive; for example, a polyanion compound may be added for providing gradual release of bFGF, as disclosed in Japanese Kokai Publication Hei-08-325160. When bFGF is used as the angiogenesis promoting factor and another hydrogel other than gelatin, such as mentioned above, is used, the hydrogel preferably has a low isoelectric point from the bFGF retention viewpoint.

The duration of the angiogenesis promoting effect can be varied by varying the biodegradability and water content of the hydrogel to be used.

From the viewpoint of gradual release of the angiogenesis promoting growth factor, the water content of the hydrogel after swelling in water is preferably not lower than 80%, more preferably 85 to 99%, still more preferably 90 to 98%.

The water content w of the hydrogel can be determined in the following manner.

When the hydrogel is in a granular form, the water content is calculated as follows:

$$w \; [\%] \; = \; 100 \; \times \; (V_W - V_D)/V_W$$

where $V_D$ is the volume of the hydrogel after lyophilization and tapping (placing the hydrogel in a measuring cylinder and giving a physical stimulus to the measuring cylinder to attain close packing of the hydrogel) and $V_W$ is the volume of the hydrogel after 24 hours of swelling in water at 37°C, followed by tapping. The method of measuring $V_D$ and $V_N$ comprises, for example, repeating the procedure of dropping the measuring cylinder from a height of about 5 cm and reading the volume when there is no more change in volume. As for $V_W$, the dropping procedure mentioned above is repeated about 10 times and, when there is no more change in volume after 30 minutes of standing, the volume is determined by reading it on the scale, since some time may be required for the hydrogel to settle in water.

When the hydrogel is not granular, the water content is calculated as follows:

$$w \; [\%] \; = \; 100 \; \times \; (W_W - W_D)/W_W$$

where $W_W$ is the weight (wet weight) of the hydrogel after 24 hours of swelling in water at 37°C, followed by removal of that portion of water adhering to the hydrogel and that interstitial water portion which occurs among hydrogel particles by suction filtration, centrifugation or the like, and $W_D$ is the weight (dry weight) of the hydrogel after the subsequent drying until there is found no more change in weight.

As a specific method of determining the wet weight, there may be mentioned, for example, the method comprising placing the hydrogel on a glass filter, suctioning the hydrogel using an aspirator, constructing a weight decrease curve showing the relationship between the suction time and the weight, and measuring the weight after the lapse of a suction time when the gradient of the curve becomes slight. The method of drying is not particularly restricted provided that a constant weight can be attained, but the method comprising drying at a temperature of not lower than 105°C under atmospheric pressure can be employed in a simple and easy manner without requiring any particular apparatus. In cases where decomposition or other phenomena may be induced by drying at such a temperature, the technique of drying under reduced pressure at a lower temperature, such as lyophilization, can also be employed.

The hydrogel to be used in the practice of the invention is obtained by rendering the above-mentioned raw material insoluble in water by crosslinking when that material is soluble in water. Such a method of crosslinking as heat treatment, ultraviolet or gamma ray irradiation, or reaction with a curing agent maybe utilized according to the origin, properties and/or the like of the material.

The curing agent is not particularly restricted but, when gelatin, for instance, is used as the hydrogel material, it includes such inorganic compounds as salts containing a polyvalent metal ion such as the aluminum or ferric ion; aldehydes such as glutaraldehyde and formalin, carbodiimides such as 1-ethyl-3-(3-dimethylaminopropyl)carbodiimide hydrochloride and 1-cyclohexyl-3-(2-morpholinoethyl)carbodiimide metho-p-toluenesulfonate, epoxy compounds such as epichlorohydrin and butanediol diglycidyl ether, isocyanates, typically hexamethylene diisocyanate, acid anhydrides and other organic compounds.

As for the form of the hydrogel to be used in the practice of the invention, the hydrogel may be in a columnar, sheet-like, disk-like, spherical, granular, or amorphous form, for instance. In view of the ease of injection into the sponge sheet, however, a spherical, granular or amorphous form is preferred.

As for the size of the hydrogel, the average particle size thereof is preferably 10 to 200 $\mu$m, more preferably 20 to 150 um.

The sponge sheet to be used in the practice of the invention is a porous sheet for immobilizing the angiogenesis promoting growth factor-containing hydrogel and for holding the cells with megalin expressed on the surface.

The raw material of the sponge sheet includes those raw materials of the hydrogel mentioned above and, further, polyesters such as polyglycolic acid, polylactic acid, polycaprolactone, polydioxanone, polyhydroxyacetic acid and polyhydroxyvaleric acid, polytrimethylene carbonate, poly($\alpha$-cyanoacrylate) and like bioabsorbable materials, and polyurethanes, among others. In the practice of the present invention, these materials may be used singly or a plurality of them may be used in combination, or they may be used after further modification with another compound.

Among the sponge sheet materials mentioned above, collagen, polyglycolic acid, polylactic acid and the like are preferred in view of the practical use thereof as biocompatible materials in the art. In particular, collagen sponge sheets are preferably used since live cells well adhere thereto.

The sponge sheet to be used in the practice of the invention has a porous structure. In view of the ease of penetration of new blood vessels and the ease of supply of oxygen and nutrients to the cells injected, it preferably has a completely open pore structure in which pores are intercommunicated.

The mean pore size thereof is 60 to 400 $\mu$m, more preferably 70 to 150 um.

Megalin is an endocytosis receptor playing a central role in the protein metabolizing function of proximal tubular cells and cDNA cloning thereof was successfully made in rats and the full-length primary structure was analyzed by Saito et al., who are the present inventors (Proc. Natl. Acad. Sci. USA 91, pp. 9725-9729 (1994)). Thereafter, human megalin/gp330 was cloned and the full-length primary structure thereof was analyzed by Hjalm G et al. (Eur. J. Biochem. 239, pp. 132-137 (1996)). Human megalin is estimated to comprise 4655-aa (molecular weight 519, 636) . As is known, it is composed of an N terminus (25-aa), an extracellular region (4398-aa), a transmembrane domain (23-aa) and a C-terminal intracellular region (209-aa), the extracellular region has cystein-rich regions of three types, and the megalin gene belongs to the LDL receptor gene family.

Megalin is a polyfunctional endocytosis receptor reportedly binding to such enzymes and enzyme inhibitors as PAI-1, PAI-1 urokinase, PAI-1-tPA, prourokinase, lipoprotein lipase and aprotinin, such vitamin binding proteins as vitamin D binding protein and retinol binding protein, such apolipoproteins as apolipoprotein B and apolipoprotein E, such basic polypeptides as aminoglycosides and polymyxin B, and such low-molecular-weight proteins and hormones as parathyroid hormone, insulin, $\beta$2-m, epidermal growth factor, prolactin, lysozyme and cytochrome c, among others.

It has been established that, in humans, for instance, megalin is expressed not only on the surface of proximal tubular epithelial cells but also on the surface of parathyroid (parathyroid gland) cells, epithelial cell layers in placental intervillous spaces, epididymal epithelial cells, type II alveolar epithelial cells, mammary epithelial cells, thyroid gland vesicular cells, ocular ciliary body epithelial cells, and so forth. Thus, these cells may be mentioned as megalin expressing cells. Among the cells mentioned above, proximal tubular epithelial cells collected from the patient's own remaining kidney are most preferably used as the megalin expressing cells.

As the cells with megalin expressed on the surface thereof, which are to be used in the practice of the present invention, there may be mentioned, in addition to the above-mentioned megalin expressing cells from the patient or some other person, cells capable of high level expression of megalin as derived from megalin expressing cells from the patient or some other person by gene transfer, cells capable of megalin expression as derived from non-megalin expressing cells from the patient or some other person by gene transfer and, furthermore, megalin expressing cells derived from embryonic cells, stem cells or the like by inducing differentiation. Usable as the method of gene transfer are those physical, chemical or biological methods which are known in the art. Usable as the method of differentiation induction are, for example, the method comprising adding a differentiation inducing substance, the method comprising cocultivation with other cells, and the method comprising varying such environmental factors as temperature, pressure, pH or/and osmotic pressure.

Among the above-mentioned cells with megalin expressed on the surface, the patient's own cells are preferred from the viewpoint of the excludability of cells implanted into the body. Furthermore, they are preferably human proximal tubular epithelial cells and/or human proximal tubular epithelial cells with megalin expressed at a high level as a result of gene transfer.

As for the non-megalin expressing cells, highly phagocytic macrophage-like cells derived from the patient's own peripheral blood are preferred from the easy technical skill viewpoint.

The expression of megalin is preferably caused by gene manipulation.

Now, the method of constructing an intra-corporeal artificial kidney having the function of metabolizing protein according to the invention is described.

The intra-corporeal artificial kidney according to the invention is constructed using a sponge sheet with an angiogenesis promoting growth factor-containing hydrogel injected therein as well as cells with megalin expressed on the surface thereof. Preferably, the method of constructing an artificial kidney comprises the step of injecting an angiogenesis promoting growth factor-containing hydrogel into a sponge sheet, and the step of injecting cells with megalin expressed on the surface into the implanted sponge sheet, wherein the sponge sheet is to be implanted subcutaneously.

The hydrogel can be prepared by any of the methods known in the art, without any particular restriction. For example, there may be mentioned the method which utilizes the phenomenon of physical intermolecular aggregation through hydrogen bonds, ionic bonds, coordination bonds, etc., the method which causes chemical crosslinking using a crosslinking agent, and the method which causes crosslinking by light or radiation. The method of hydrogel molding (forming) is not particularly restricted but, for example, the emulsion method, molding method, or spray drying method can be utilized for preparing granular hydrogels.

For causing the angiogenesis promoting growth factor to be contained in the hydrogel, any method can be used without any particular restriction provided that the biological activity of the angiogenesis promoting growth factor is not much impaired. For example, there may be mentioned the method which comprises drying the hydrogel after preparation thereof and impregnating the same with a solution containing the growth factor, or the method which comprises impregnating the hydrogel as prepared with a growth factor-containing solution without drying the hydrogel or, further, the method which comprises causing the growth factor to occur in the starting solution for preparing the hydrogel.

The angiogenesis promoting growth factor-containing hydrogel can be injected into the sponge sheet using a syringe, for instance.

For immobilizing the angiogenesis promoting growth factor-containing hydrogel and for holding the cells with megalin expressed on the surface, the sponge sheet is implanted into a living body subcutaneously or some other site for utilization thereof. The implantation of the sponge sheet may be made after injection of the angiogenesis promoting growth factor-containing hydrogel thereinto, or the sponge sheet as such may be implanted, followed by injection of the hydrogel thereinto.

The intra-corporeal artificial kidney of the invention can be constructed by injecting the cells with megalin expressed on the surface into the sponge sheet implanted into a living body with the angiogenesis promoting growth factor-containing gel injected therein.

The cells with megalin expressed on the surface thereof can be injected into the sponge sheet using a syringe or a microcapillary, for instance.

The timing of the injection of the cells with megalin expressed on the surface is not particularly restricted but, for securing the adhesion and propagation of the live cells with megalin expressed on the surface, it is desirable that the injection of the cells be made after the lapse of a necessary number of days for angiogenesis after implantation of the sponge sheet with the angiogenesis promoting growth factor-containing hydrogel injected therein or after injection of the growth factor-containing hydrogel into the sponge sheet implanted in advance. The necessary number of days for angiogenesis depends on the angiogenesis promoting growth factor employed and on the properties of the hydrogel. When a bFGF-containing, alkali-treated gelatin gel is used, for instance, the number of days is generally 3 to 8.

BRIEF DESCRIPTION OF THE DRAWINGS

Fig. 1 is a graphic representation of the data concerning the uptake of $^{125}$I-labeled β2-m in several tissues in Example and Comparative Example. In the figure, the following symbols are used: transpl.: Example; cont.: Comparative Example; T: transplanted cell phyma; L: liver; P: lung; H: heart; S: skeletal muscle.

Fig. 2 is a presentation of the results of SDS-polyacrylamide gel electrophoretic analysis of $^{125}$I-labeled β2-m incorporated into transplanted cells. In the figure, the lanes are respectively for the following samples:

Lane 1: $^{125}$I-labeled β2-m used for intraperitoneal administration.
Lane 2: Homogenate of a transplanted cell phyma recovered after testing.
Lane 3: Blood collected from a cell transplanted mouse after testing.

Fig. 3 is a graphic representation of the results of radioisotope counting of trichloroacetic acid (TCA)-caused blood precipitates in Example and Comparative Example. In the figure, the following terms are used:

control group for Comparative Example;
transplanted group for Example.

BEST MODES FOR CARRYING OUT THE INVENTION

(Example)

(1) Preparation of bFGF-containing gelatin gel particles

bFGF-Containing gelatin gel particles were prepared by the method of Tabata et al. (Tabata, Y., et al., J. Biomateri. Sci. Polymer Edn., 10: 957-968, 1999). Thus, 10 mL of a 10 weight % aqueous solution of alkali-treated gelatin (isoelectric point 5.0) (product of Nitta Gelatin), preheated to 40°C, was admixed with 25 µL of a 25 weight % aqueous solution of glutaraldehyde, and the mixture was added dropwise to 375 mL of olive oil at 40°C with stirring at 425 rpm to form a w/o emulsion. The stirring was then continued at 25°C for 24 hours to chemically crosslink the gelatin. Thereto was added 100 mL of acetone, and the resulting particles were collected by centrifugation (4°C, 3, 000 rpm, 5 minutes) and washed 5 times by centrifugation in acetone. The particles after washing were maintained in 100 mL of a 100 mM aqueous glycine solution containing 0.1 weight % of Tween 80 at 37°C for 1 hour to block remaining unreacted aldehyde groups of glutaraldehyde origin. The thus-obtained crosslinked gelatin particles were washed twice by centrifugation in distilled water, then lyophilized, and sterilized with ethylene oxide gas. After 24 hours of swelling in water at 37°C, the gelatin gel particles showed a water content of 95%. The particle sizes (diameters) were measured under an optical microscope and found to be 60 to 130 µm. A 10 mg/mL aqueous solution (10 µL) of recombinant bFGF with an isoelectric point of 9.6 (product of Kaken

Pharmaceutical Co.) was added dropwise onto a 2-mg portion of the dry gelatin gel particles obtained in the above manner, and the whole was allowed to stand at 25°C for 1 hour to give bFGF-containing gelatin gel particles.

(2) Preparation of a gel sponge sheet

A 0.3% atherocollagen hydrochloric acid solution (pH 3.0) was stirred at 1,800 to 2,000 rpm for 60 minutes using a refrigerated homogenizer. The foamed solution was cast into a mold, rapidly frozen at -40°C, lyophilized for 48 hours, and further dried at 105°C under reduced pressure for 24 hours. Then, crosslinking was carried out in a 0.2% glutaraldehyde solution in 0.05 M acetic acid at 4°C for 24 hours, and the molding was washed with phosphate-buffered physiological saline (PBS) (pH 7.4) and immersed in a 15% aqueous ethanol solution. The molding was rapidly frozen at -135°C, then lyophilized (48 hours), and sterilized with ethylene oxide gas to give a collagen sponge sheet.

(3) Subcutaneous transplantation of megalin expressing cells

The bFGF-containing gelatin gel particles obtained as described above under (1) were suspended in 0.15 mL of PBS, and the suspension was injected into the collagen sponge sheet (1 x 1 x 0.5 cm) obtained as described above under (2) using a 1-mL syringe (with a 23G needle). The sponge sheet was implanted into a 5-week-old female nude mouse (BALB/cA Jcl-nu; CLEA Japan) subcutaneously on the dorsal side under anesthesia by diethyl ether inhalation.

One week later, when angiogenesis in the sponge sheet was pathologically confirmed, 0.15 mL of a cell suspension prepared by suspending the cell derived from rat yolk sac epithelial cancer cell (L2 cells) cultivated in Dulbecco's Modified Eagle medium (LIFE TECHNOLOGIES, GIBCO BRL, Rockville, MD, USA) supplemented with 10% (vol/vol) neonatal bovine serum and now carrying megalin expressed on the surface thereof in PBS (1 x $10^7$ cells/mL) was injected into the sponge sheet using a 1-mL syringe (with a 23G needle) under anesthesia of the mouse. Two weeks after cell injection, when the cell phyma had a longest diameter of not shorter than 2 cm, both kidneys of the mouse were surgically excised from the dorsal side under anesthesia to bring about a renal insufficiency state in the mouse. No metastasis of the L2 cells transplanted was observed.

(Comparative Example)

The same procedure as in the above example was followed except that the cell suspension was not injected but PBS alone was injected into the collagen sponge sheet in the stage (3) in the above Example.

(Test Example)

The mice in a renal insufficiency state as obtained in the manner described above in Example and Comparative Example were subjected to the following tests.

(1) β2-Microglobulin (product of Oriental Yeast Co.) was $^{125}$I-labeled using IODOGEN (product of Pierce). The iodination gave a specific activity of 6 x $10^3$ cpm/ng protein. The $^{125}$I-labeled β2-m was diluted with PBS containing 0.5% bovine serum albumin (BSA) to a concentration of 11 ng/μL. The $^{125}$I-labeled β2-m (2.8 μg/250 μL) was intraperitoneally administered to each of 15 mice in the renal insufficiency state mentioned above and, for evaluating the mice for the items mentioned below, 5 mice were sacrificed by general perfusion with physiological saline at 3, 6 or 14 hours after administration of $^{125}$I-labeled β2-m.

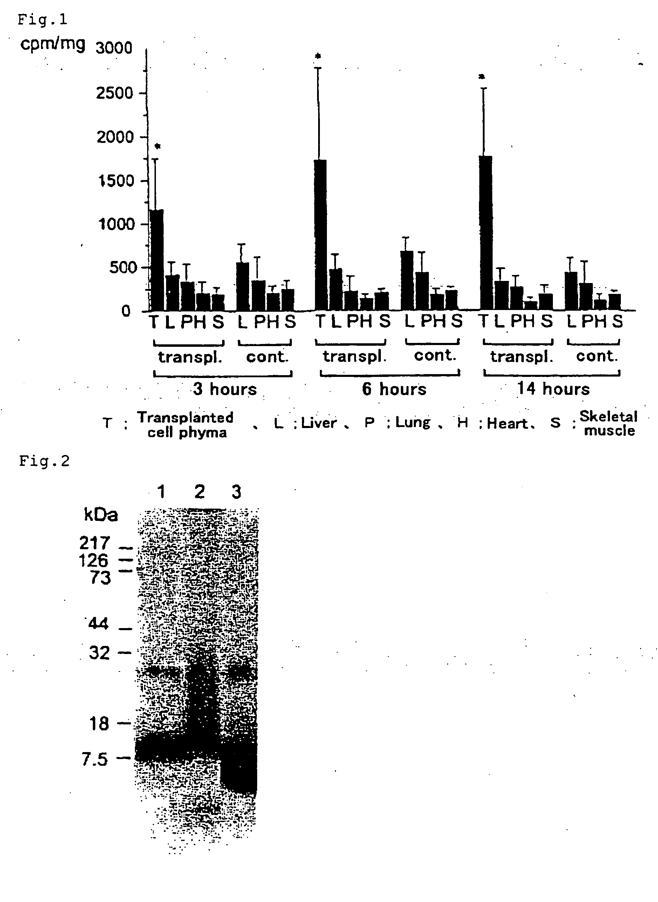

(2) The transplanted cell phyma, heart, lung, liver and skeletal muscle were collected from each mouse after sacrifice, and the tissues/organs were weighed and subjected to gamma ray counting in Eppendorf tubes. The results thus obtained are shown in Fig. 1. Fig. 1 is a graphic representation of the data concerning the uptake of $^{125}$I-labeled β2-m in the tissues in Example and Comparative Example. The ordinate denotes the $^{125}$I count per unit tissue weight. The $^{125}$I counts in the transplanted cells were always significantly (*: p < 0.05) higher than those in the heart, lung, liver and skeletal muscle, irrespective of the time of measurement. On the other hand, the $^{125}$I counts in the heart, lung, liver and skeletal muscle failed to show any significant difference between Example and Comparative Example, irrespective of the time of measurement. These results indicate that $^{125}$I-labeled β2-m had been efficiently incorporated into the transplanted cells.

(3) Furthermore, the transplanted cell phyma was homogenized in Laemmli sample buffer supplemented with 2% β-mercaptoethanol using ULTRA TURRAX (IKA LABORTECHNIK, Staufen, Germany), and the homogenate was subjected to SDS-polyacrylamide gel electrophoretic analysis. The results thus obtained are shown in Fig. 2.

Fig. 2 is a presentation of the results of SDS-polyacrylamide gel electrophoretic analysis of $^{125}$I-labeled (β2-m incorporated into the transplanted cells. Comparison with the results for $^{125}$I-labeled β2-m used for intraperitoneal administration reveals that the transplanted cells contain the $^{125}$I-labeled β2-m monomer and its decomposition products. On the other hand, in the blood sample from a cell transplantation recipient mouse, there were observed, in addition to the decomposition products, β2-m concatemers in larger amounts than in the transplanted cells. These results can be said to indicate that the radioisotope count in the transplanted cells are not due to impurities in the blood but due to decomposition of β2-m incorporated into the transplanted cells.

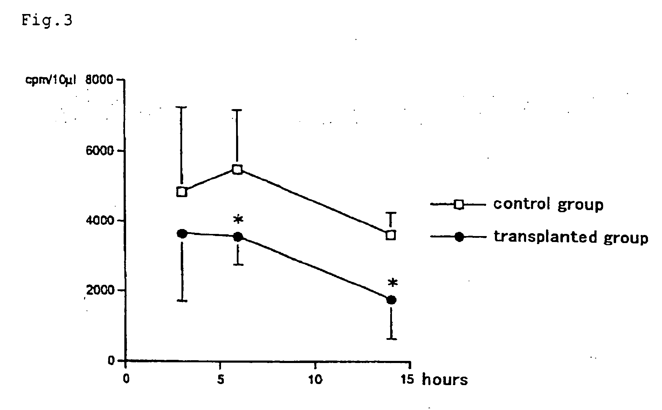

(4) Furthermore, blood was collected from each mouse after sacrifice, a 10-μL portion of the blood sample was mixed with 415 μL of PBS containing 1% BSA, the mixture was then mixed with 75 μL of 100% trichloroacetic acid (hereinafter, TCA) to precipitate the undecomposed protein. The precipitate protein was collected by centrifugation and counted using a gamma counter, and the level of undecomposed $^{125}$I-labeled β2-m in blood was thereby evaluated. The results thus obtained are shown in Fig. 3.

Fig. 3 is a graphic representation of the results of radioisotope counting of TCA-caused blood precipitates in Example and Comparative Example. At 6 and 14 hours after administration of $^{125}$I-labeled β2-m, the radioisotope counts in the TCA precipitates from blood decreased significantly (*: $p < 0.05$) in Example as compared with Comparative Example. This indicates that $^{125}$I-labeled β2-m was decomposed in larger amounts in Example as compared with Comparative Example, namely that the transplanted cells were producing the function of metabolizing protein.

INDUSTRIAL APPLICABILITY

The artificial kidney having a function of metabolizing protein, according to the invention, can function *in vivo* and is useful in improving the renal insufficiency patient's QOL.

**Claims**

1. An intra-corporeal artificial kidney having a function of metabolizing protein
   which comprises, as constituent elements, a sponge sheet with an angiogenesis promoting growth factor-containing hydrogel injected therein as well as cells with megalin expressed on the surface
   wherein the mean pore size of said sponge sheet is 60 to 400 μm.

2. The artificial kidney according to Claim 1, wherein the angiogenesis promoting growth factor is a basic fibroblast growth factor (bFGF).

3. The artificial kidney according to Claim 2, wherein the bFGF is human bFGF produced by genetic engineering techniques.

4. The artificial kidney according to Claim 1, wherein the hydrogel is a gelatin gel.

5. The artificial kidney according to Claim 4, wherein the gelatin gel is mainly composed of a gelatin species having an isoelectric point of 4.5 to 5.5.

6. The artificial kidney according to Claim 1, wherein the sponge sheet is made of collagen.

7. The artificial kidney according to Claim 1, wherein the cells with megalin expressed on the surface are human proximal tubular epithelial cells and/or human proximal tubular epithelial cells with megalin expressed on the surface at a high level by means of gene transfer.

8. The artificial kidney according to Claim 1, wherein the expression of megalin is a result of gene manipulation.

9. A use of a sponge sheet with an angiogenesis promoting growth factor-containing hydrogel injected therein as well as cells with megalin expressed on the surface,
   for preparing an intra-corporeal artificial kidney to metabolize protein,
   wherein the mean pore size of said sponge sheet is 60 to 400 μm.

10. The use according to Claim 9, which comprises the step of injecting an angiogenesis promoting growth factor-containing hydrogel into a sponge sheet, and the step of injecting cells with megalin expressed on the surface into the implanted sponge sheet, wherein the sponge sheet is to be implanted subcutaneously.

11. The use according to Claim 9, wherein the angiogenesis promoting growth factor is bFGF.

12. The use according to Claim 11, wherein the bFGF is human bFGF produced by genetic engineering techniques.

13. The use according to Claim 9, wherein the hydrogel is a gelatin gel.

14. The use according to Claim 13, wherein the gelatin gel is mainly composed of a gelatin species having an isoelectric point of 4.5 to 5.5.

15. The use according to Claim 9, wherein the sponge sheet is made of collagen.

16. The use according to Claim 9, wherein the cells with megalin expressed on the surface are human proximal tubular epithelial cells and/or human proximal tubular epithelial cells with megalin expressed on the surface at a high level by means of gene transfer.

17. The use according to Claim 9, wherein the expression of megalin is a result of gene manipulation.

**Patentansprüche**

1. Intrakorporale, künstliche Niere mit Proteinmetobilisierungsfunktion,
   die als Bestandteile eine schwammartige Folie mit darin injiziertem Hydrogel, welches einen Angiogenese-fördernden Wachstumsfaktor enthält, sowie Zellen mit auf der Oberfläche exprimiertem Megalin umfasst,
wobei die durchschnittliche Porengröße der schwammartigen Folie 60 bis 400 μm beträgt.

2. Künstliche Niere gemäß Anspruch 1, wobei der Angiogenese-fördernde Wachstumsfaktor ein basischer Fibroblastenwachstumsfaktor (bFGF) ist.

3. Künstliche Niere gemäß Anspruch 2, wobei der bFGF ein menschlicher bFGF ist, der durch gentechnologische Techniken hergestellt wird.

4. Künstliche Niere gemäß Anspruch 1, wobei das Hydrogel ein Gelatinegel ist.

5. Künstliche Niere gemäß Anspruch 4, wobei das Gelatinegel vorwiegend aus einer Gelatineart mit einem isoelektrischen Punkt von 4,5 bis 5,5 besteht.

6. Künstliche Niere gemäß Anspruch 1, wobei die schwammartige Folie aus Kollagen hergestellt wird.

7. Künstliche Niere gemäß Anspruch 1, wobei die Zellen mit auf der Oberfläche exprimiertem Megalin menschliche, proximale Tubulusepithelzellen und/oder menschliche, proximale Tubulusepithelzellen mit Megalin sind, welches auf der Oberfläche mittels Gentransfer in hoher Konzentration exprimiert wird.

8. Künstliche Niere gemäß Anspruch 1, wobei die Megalinexpression eine Folge von Genmanipulation ist.

9. Verwendung einer schwammartigen Folie mit einem darin injizierten Hydrogel, das einen Angiogenese-fördernden Wachstumsfaktor enthält, sowie Zellen mit auf der Oberfläche exprimiertem Megalin, zur Herstellung einer intrakorporalen, künstlichen Niere zur Proteinmetabolisierung, wobei die durchschnittliche Porengröße der schwammartigen Folie 60 bis 400 μm beträgt.

10. Verwendung gemäß Anspruch 9, die einen Injektionsschritt eines Hydrogels, das einen Angiogenese-fördernden Wachstumsfaktor enthält, in eine schwammartige Folie sowie einen Injektionsschritt von Zellen mit auf der Oberfläche exprimiertem Megalin in die implantierte schwammartige Folie umfasst, wobei die schwammartige Folie subkutan implantiert werden soll.

11. Verwendung gemäß Anspruch 9, wobei der Angiogenese-fördernde Wachstumsfaktor bFGF ist.

12. Verwendung gemäß Anspruch 11, wobei der bFGF ein menschlicher bFGF ist, der durch gentechnologische Techniken hergestellt wird.

13. Verwendung gemäß Anspruch 9, wobei das Hydrogel ein Gelatinegel ist.

14. Verwendung gemäß Anspruch 13, wobei das Gelatinegel hauptsächlich aus einer Gelatineart mit einem isoelektrischen Punkt von 4,5 bis 5,5 besteht.

15. Verwendung gemäß Anspruch 9, wobei die schwammartige Folie aus Kollagen hergestellt wird.

16. Verwendung gemäß Anspruch 9, wobei die Zellen mit auf der Oberfläche exprimiertem Megalin menschliche, proximale Tubulusepithelzellen und/oder menschliche, proximale Tubulusepithelzellen mit Megalin sind, welches auf der Oberfläche mittels Gentransfer in hoher Konzentration exprimiert wird.

17. Verwendung gemäß Anspruch 9, wobei die Megalinexpression eine Folge von Genmanipulation ist.

**Revendications**

1. Rein artificiel intracorporel ayant une fonction de métaboliser les protéines
qui comprend, comme éléments constitutifs, une feuille d'éponge avec un hydrogel contenant un facteur de croissance favorisant l'angiogenèse injecté dans celle-ci ainsi que des cellules avec de la mégaline exprimée sur la surface,
où la taille de pore moyenne de ladite feuille d'éponge est 60 à 400 μm.

2. Rein artificiel selon la revendication 1,
où le facteur de croissance favorisant l'angiogenèse est un facteur de croissance des fibroblastes basique (bFGF).

3. Rein artificiel selon la revendication 2,
où le bFGF est un bFGF humain produit par des techniques de génie génétique.

4. Rein artificiel selon la revendication 1,
où l'hydrogel est un gel de gélatine.

5. Rein artificiel selon la revendication 4,
où le gel de gélatine est composé principalement d'une espèce de gélatine ayant un point isoélectrique de 4,5 à 5,5.

6. Rein artificiel selon la revendication 1,
où la feuille d'éponge est faite de collagène.

7. Rein artificiel selon la revendication 1,
où les cellules avec de la mégaline exprimée sur la surface sont des cellules épithéliales tubulaires proximales humaines et/ou des cellules épithéliales tubulaires proximales humaines avec de la mégaline exprimée sur la surface à un haut niveau par transfert génique.

8. Rein artificiel selon la revendication 1, où l'expression de mégaline résulte de manipulation génique.

9. Utilisation d'une feuille d'éponge avec un hydrogel contenant un facteur de croissance favorisant l'angiogenèse injecté dans celle-ci ainsi que des cellules avec de la mégaline exprimée sur la surface, pour préparer un rein artificiel intracorporel pour métaboliser les protéines, où la taille de pore moyenne de ladite feuille d'éponge est 60 à 400 μm.

10. Utilisation selon la revendication 9, qui comprend l'étape d'injection d'un hydrogel contenant un facteur de croissance favorisant l'angiogenèse dans une feuille d'éponge, et l'étape d'injection de cellules avec de la mégaline exprimée sur la surface dans la feuille d'éponge implantée, où la feuille d'éponge est destinée à être implantée par voie sous-cutanée.

11. Utilisation selon la revendication 9, où le facteur de croissance favorisant l'angiogenèse est bFGF.

12. Utilisation selon la revendication 11, où le bFGF est un bFGF humain produit par des techniques de génie génétique.

13. Utilisation selon la revendication 9, où l'hydrogel est un gel de gélatine.

14. Utilisation selon la revendication 13, où le gel de gélatine est composé principalement d'une espèce de gélatine ayant un point isoélectrique de 4,5 à 5,5.

15. Utilisation selon la revendication 9, où la feuille d'éponge est faite de collagène.

16. Utilisation selon la revendication 9, où les cellules avec de la mégaline exprimée sur la surface sont des cellules épithéliales tubulaires proximales humaines et/ou des cellules épithéliales tubulaires proximales humaines avec de la mégaline exprimée sur la surface à un haut niveau par transfert génique.

17. Utilisation selon la revendication 9, où l'expression de mégaline résulte de manipulation génique.

Fig.1

cpm/mg

T : Transplanted cell phyma , L : Liver , P : Lung , H : Heart, S : Skeletal muscle

Fig.2

Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9809582 A

- JP HEI08325160 B

**Non-patent literature cited in the description**

- **Humes, HD et al.** *Nature Biotech.,* 1999, vol. 17, 451-453
- **Saito ; Akira et al.** *44th Meeting of the Japanese Society for Dialysis Therapy,* 1999
- **Saito et al.** *Proc. Natl. Acad. Sci. USA,* 1994, vol. 91, 9725-9729

- **Hjalm G et al.** *Eur. J. Biochem.,* 1996, vol. 239, 132-137
- **Tabata, Y. et al.** *J. Biomateri. Sci. Polymer Edn.,* 1999, vol. 10, 957-968